(19) Europäisches Patentamt
European Patent Office
Office européen des brevets

(11) **EP 4 246 386 A1**

(12) **EUROPEAN PATENT APPLICATION**

(43) Date of publication:
**20.09.2023 Bulletin 2023/38**

(21) Application number: **22208797.5**

(22) Date of filing: **22.11.2022**

(51) International Patent Classification (IPC):
**G06N 20/00** (2019.01)    **G06N 3/086** (2023.01)

(52) Cooperative Patent Classification (CPC):
**G06N 3/086; G06N 20/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.03.2022 IN 202221014386**

(71) Applicant: **Tata Consultancy Services Limited Maharashtra (IN)**

(72) Inventors:
• **RAMAMURTHI, NARAYANAN**
  **600113 Chennai, Tamil Nadu (IN)**
• **KONETI, GEERVANI**
  **500081 Hyderabad, Telangana (IN)**
• **SHAIK, MASTAN VALI**
  **500081 Hyderabad, Telangana (IN)**
• **DAS, SHYAM, SUNDAR**
  **700160 Kolkata, West Bengal (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **OPTIMAL VARIABLES SELECTION FOR GENERATING PREDICTIVE MODELS USING POPULATION BASED EXHAUSTIVE REPLACEMENT TECHNIQUES**

(57)    Population based exhaustive replacement method(s) (PERM) for optimal variables selection and generation of regression models, to overcome conventional approaches, thereof is described herein. PERM initializes population based on one or more criteria, wherein one or more paths for variables/descriptors 1 to r for replacement with remaining descriptors wherein the one or more paths are updated based on relative error associated with each variable. For each combination of descriptors of r size, inter correlation of the descriptors are verified and predictive models are built. Subsets of variable with higher predictive ability are selected for substitution of initial population to obtain an updated population on which a replacement method is performed to obtain optimal set of variables. One or more variables of optimal set are randomly replaced, and perturbation can be performed on the top best subsets to converge at global optimum.

FIG. 2A

EP 4 246 386 A1

**Description**

CROSS-REFERENCE TO RELATED APPLICATIONS AND PRIORITY

The present application claims priority to Indian application no. 202221014386, filed on March 16, 2022.

TECHNICAL FIELD

The disclosure herein generally relates to search optimization techniques, and, more particularly, to optimal variables selection for generating predictive models using population based exhaustive replacement techniques.

BACKGROUND

Advancements in various sciences such as physical, life, social sciences etc., have generated large amounts of data and there is great interest to make use of these data for the creation of new knowledge, as it is expected to improve the quality of human life. The quest for the new knowledge that includes insights, rules, alerts, predictive models etc. and its associated positive impact on humanity, have created an urgent need for the development of efficient data analytics techniques and technologies such as high performance computing, cloud computing etc., which can handle large amounts of data. Variable selection methods are one such data analytics approach that is applied to the selection of a subset of variables (X) from a large pool of variables based on various statistics measures. The selected variables can be used for the development of prediction models for a dependent variable (Y), when used with modelling techniques such as multiple linear regression, nonlinear regression, etc. The variables selection can be accomplished using a random or exhaustive search technique. The random approach includes heuristic methods such as ant colony, particle swarm optimization, genetic algorithm, and the like that are less compute intensive; however, these methods cannot guarantee an optimal solution as they fail to explore the complete problem (variable) space. Unlike a random approach, the exhaustive search approach, evaluates each possible combination and thus provides the best solution; however, it is a computationally hard problem, thus limiting its applications to the selection of smaller subsets.

Predictive regression model generation, in principle involves the following three critical steps: a) data division, b) optimal feature/variable selection from a large pool of structural features and c) model generation from the selected optimal features. Data quality and the efficiency of the above three steps determine robustness of the predictive models and their applications/business impact. For example, failures of drug candidates in late stages can be addressed using reliable and easily applicable predictive regression ADMET models [Absorption, Distribution, Metabolism, Excretion and Toxicity]. As these computational models rationalize experimental observations, offer potential for virtual screening applications, and consequently can help in reducing time and cost of the drug discovery and development process, consequently, have wide applications within pharmaceutical industry. The generation of predictive ADMET models based on structural features of drugs and drug candidates typically involves three critical steps, discussed earlier. The variable selection step enables researchers to a) derive rules/alerts that can be used for improving research outcomes, and b) provide the best sub-set of variables to generate robust predictive models that are applicable in virtual screening of drug candidates even before they are produced in laboratory.

The use of large number of descriptors/physico-chemical properties that are indicative of molecular structural features is becoming more common and selection of optimal numbers of non-correlated features (variable selection) from a large number of features is a computationally intensive step. Systematic search methods are expected to provide the best possible solutions. For instance, conventional approach such as Replacement Method (RM) involves selecting an initial set of descriptors of size 'r' at random whose inter correlation among the selected subset of descriptors is less than a user defined threshold, and then for each path 'l', which ranges from '1' to 'r', l^th descriptor is replaced with all remaining descriptors and objective function for each updated subset is calculated. Thereafter, a subset (referred as optimal subset) is chosen from the above generated subsets having maximum or minimum objective function based on the problem description. Following this, standard errors/deviation of the descriptors of the optimal subset are computed to choose a descriptor with highest standard error to replace next until one or more stopping criterion is met.

Another conventional approach referred as 'Modified Replacement Method' or MRM, wherein this follows similar algorithm as RM but in each step the descriptor with largest error is substituted even if that substitution results in a lower objective function. In MRM, the stopping criteria is frequency of an optimal set and number of steps.

Yet another conventional approach referred as 'Enhanced Replacement Method (ERM)' which is a combination of conventional methods RM and MRM in which MRM is performed in between RM. In this algorithm, RM method is first performed, and the output of RM is set as input for MRM and the output of MRM is again used as input for the RM

Yet further conventional approach includes RM and ERM First Step Modification (RMfsm and ERMfsm). Authors of this conventional approach proposed many versions of the RM and ERM, which can contain one or more of the below steps (a) First step modification: in this only one path is selected to replace based on relative standard deviation rather than all possible 'd' paths; (b) Arbitrary first step: the descriptor to be replaced is selected randomly; (c) Using more than one starting initial subsets; (d) Selecting initial subset with high standard deviation, and the like.

Yet another conventional approach is ERM with Genetic Algorithm (GA). Authors of this conventional approach proposed various strategies for integration of genetic algorithm and enhanced replacement algorithm. They are: (a) GA with mutation operator position determined by rsd instead of a random selection; (b) GA with crossover operator position determined by rsd instead a random selection; (c) GA with both modified mutation and modified crossover operator; and (d) ERM with an initial population (ERMp) similar to GA.

Though these conventional approaches performed better in some way or the other to produce near satisfactory results, however, they have their own limitations such as unrealistic computing time, particularly when the number of variables to be selected are higher and consequently, wherein optimal solution is not certain and suffer from slower convergence of the solution, lower predictive capability of the models and the like. Because of the unrealistic computing time required, selection and generation of models with larger number of variables becomes a challenging task, thereby, minimizing the applications of the models with pharmaceutical industry. In other words, the conventional approaches as known in the art are computationally intensive and consequently, may not be employed regularly in solving real world problems within an industry (e.g., pharmaceutical industry) due to lack of needed resources and time, when the end-user needs to build models based on a large number of variables.

SUMMARY

Embodiments of the present disclosure present technological improvements as solutions to one or more of the above-mentioned technical problems recognized by the inventors in conventional systems. For example, in one aspect, there is provided processor implemented population based exhaustive replacement method for selecting one or more optimal variables from a set of unique variables and generating predictive models thereof. The method comprises: (i) inputting, via one or more hardware processors, a set of physico-chemical properties X derived from chemical structures of drugs and drug like chemical compounds, a biological response $Y$ associated thereof and a size of variables set r; (ii) initializing a population $S_{pop}$ comprising matrix subsets $\{X_1, X_2, ... X_{pop}\}$ of variables selected from a filtered set of variables $X_f$ of size $n$, wherein $X_f$ is derived from a set of variables X, wherein $S_{pop}$ is initialized based on one or more pre-defined criteria, wherein pop represents size of the population, and wherein each matrix subset $X_i$ from $S_{pop}$ comprises variables that are unique from each other and is of the size of variables set r; (iii) selecting at least one subset $X_i$ from $S_{pop}$ and at least one path $P_l$ of the at least one selected subset $X_i$, wherein the at least one selected path $P_l$ comprises a variable $v_q$ to be replaced, wherein each variable $v_q$ comprised in the at least one selected path $P_l$ is a vector of size m describing a property of input chemical compounds, and wherein m represents a number of chemical compounds used for building predictive models; (iv) replacing the variable $v_q$ from at least one subset Xiwith remaining $(n - r)$ variables of the set of variables $X_f$ to obtain a set of modified subsets $X_i' \{X_{i1}', X_{i2}', ..., X_{i(n-r)}'\}$, wherein each of the modified subsets $X_{ij}'$ comprises replaced variables for the at least selected path $P_l$, and wherein size of the set of modified subsets $X_i'$ is of $(n - r)$; (v) generating a predictive model for each of the modified subsets $X_{ij}'$, and calculating an objective function thereof to obtain a first set of predictive models $M_{i,rm}$ and associated objective functions $OF_{i,rm}$, wherein the first set of predictive models $M_{i,rm}$ are generated based on the biological response $Y$ and each of the modified subsets $X_{ij}'$ variable vectors of a set of input chemical compounds; (vi) identifying an optimal modified subset of replaced variables $X_i^{optimal}$ from the set of modified subsets $X_i'$ based on an optimal objective function associated with an optimal predictive model $M_i^{optimal}$ being identified from the first set of predictive models $M_{i,rm}$; (vii) updating the at least one selected path $P_l$ for the optimal modified subset of replaced variables $X_i^{optimal}$, wherein the steps (iv) till (vii) are iteratively performed until one or more predefined criteria are met to obtain an optimal population $S_{pop}^{optimal}$; (viii) identifying an optimal element $X_{optimal}$ of $S_{pop}^{optimal}$, wherein $X_{optimal}$ comprises an optimal objective function amongst objective functions comprised in other $X_i^{optimal}$ ; (ix) performing an exhaustive search on a pool of variable $X_{pool}$ created using the optimal population $S_{pop}^{optimal}$ to obtain a set of variable subsets $S_x$, wherein each element of $S_x$ comprises set of $r$ variables; (x) generating the predictive model for each element of $S_x$ and calculating the objective function thereof to obtain a second set of predictive models $M_x$ and associated objective functions $OF_x$; (xi) identifying a pop number of optimal elements from $S_x$ to update the optimal population $S_{pop}^{optimal}$ and to obtain an updated population $S_{es}$; (xii) identifying an optimal element $X_{optimal,es}$ amongst elements $X_{es}$ comprised in the updated population $S_{es}$, and comparing an objective function of $X_{optimal,es}$ with an objective function of the identified optimal element $X_{optimal}$ for updation of the identified optimal element $X_{optimal}$; (xiii) randomly replacing variables of the updated population $S_{es}$ to obtain a perturbed set of population $S_P$; and (xiv) generating one or more predictive models based on the selected optimal subset of variables $X_{optimal}$ .

In an embodiment, the one or more predefined criteria comprise: (i) subsets in which variables having inter correlation amongst each other below a first predefined threshold, (ii) subsets whose variables are correlated with the biological response Y above a second defined threshold or a system generated dynamic threshold; (iii) seeded subsets having variables whose sizes are less than a current subset size and (iv) a user defined preferences for the set of variables $X$.

In an embodiment, the at least one selected path $P_l$ is identified based on a relative error value associated with the variable $v_q$.

In an embodiment, the steps (ii) till (xiii) are iteratively performed either sequentially or in parallel across multiple processors threads.

In an embodiment, the steps (iv) till (vii) are iteratively performed until the one or more predefined criteria are met, and wherein the one or more predefined criteria further comprise at least one of (a) a predefined number of iterations, (b) frequency of the matrix subset of variables, (c) the optimal objective function reaching a predetermined value, and (d) an improvement in the objective function in subsequent iterations reaching a predefined saturation value or machine epsilon.

In an embodiment, the steps (ix) till (xii) are performed either sequentially or in parallel across multiple processors threads.

The method may further comprise upon satisfying the one or more predefined criteria, and upon iteratively performing the steps (ii) till (xiii), generating a final optimal subset based on the identified optimal element $X_{optimal}$ for predicting biological responses of chemical compounds.

In an embodiment, the step of initializing a population $S_{pop}$ comprising matrix subsets $\{X_1, X_2, ... X_{pop}\}$ of variables selected from the filtered set of variables $X_f$ is preceded by transforming the set of variables $X$ to obtain a set of transformed variables; and filtering the set of transformed variables based on one or more statistical criteria and predefined thresholds to obtain a filtered set of variables $X_f$.

In an embodiment, the one or more predictive models comprise one or more linear regression models, one or more non-linear regression models, or one or more classification models, and wherein the one or more predictive models and the identified optimal subset $X_{optimal}$ are used for generating one or more rules or alerts.

In another aspect, a processor implemented population based exhaustive replacement system for selecting one or more optimal variables from a set of unique variables and generating predictive models thereof is provided. The system comprises a memory storing instructions; one or more communication interfaces; and one or more hardware processors coupled to the memory via the one or more communication interfaces, wherein the one or more hardware processors are configured by the instructions to: (i) receive, a set of physico-chemical properties X derived from chemical structures of drugs and drug like chemical compounds, a biological response Y associated thereof and a size of variables set r; (ii) initialize a population $S_{pop}$ comprising matrix subsets $\{X_1, X_2, ... X_{pop}\}$ of variables selected from a filtered set of variables $X_f$ of size $n$, wherein $X_f$ is derived from a set of variables X, wherein $S_{pop}$ is initialized based on one or more pre-defined criteria, wherein pop represents size of the population, and wherein each matrix subset $X_i$ from $S_{pop}$ comprises variables that are unique from each other and is of the size of variables set r; (iii) select at least one subset $X_i$ from $S_{pop}$ and at least one path of the at least one selected subset $X_i$, wherein the at least one selected path $P_l$ comprises a variable $v_q$ to be replaced, wherein each variable $v_q$ comprised in the at least one selected path $P_l$ is a vector of size m describing a property of input chemical compounds, and wherein m represents the number of chemical compounds used for building predictive models; (iv) replace the variable from at least one subset $X_i$ with remaining $(n - r)$ variables of the set of variables

$X_f$ to obtain a set of modified subsets $X'_i \{X'_{i1}, X'_{i2}, \ldots, X'_{i(n-r)}\}$, wherein each of the modified subsets $X'_{ij}$ comprises replaced variables for the at least selected path $P_l$, and wherein size of the set of modified subsets $X'_i$ is of $(n - r)$; (v) generate a predictive model for each of the modified subsets $X'_{ij}$, and calculating an objective function thereof to obtain a first set of predictive models $M_{i,rm}$ and associated objective functions $OF_{i,rm}$, wherein the first set of predictive models $M_{i,rm}$ are generated based on the biological response $Y$ and each of the modified subsets $X'_{ij}$ variable vectors of a set of input chemical compounds; (vi) identify an optimal modified subset of replaced variables $X_i^{optimal}$ from the set of modified subsets $X'_i$ based on an optimal objective function associated with an optimal predictive model $M_i^{optimal}$ being identified from the first set of predictive models $M_{i,rm}$; (vii) update the at least one selected path $P_l$ for the optimal modified subset of replaced variables $X_i^{optimal}$, wherein the steps (iv) till (vii) are iteratively performed until one or more predefined criteria are met to obtain an optimal population $S_{pop}^{optimal}$; (viii) identify an optimal element $X_{optimal}$ of $S_{pop}^{optimal}$, wherein $X_{optimal}$ comprises an optimal objective function amongst objective functions comprised in other $X_i^{optimal}$; (ix) perform an exhaustive search on a pool of variable $X_{pool}$ created using the optimal population $S_{pop}^{optimal}$ to obtain a set of variable subsets $S_x$, wherein each element of $S_x$ comprises set of r variables; (x) generate the predictive model for each element of $S_x$ and calculating the objective function thereof to obtain a second set of predictive models $M_x$ and associated objective functions $OF_x$; (xi) identify a pop number of optimal elements from $S_x$ to update the optimal population $S_{pop}^{optimal}$ and to obtain an updated population $S_{es}$; (xii) identify an optimal element $X_{optimal,es}$ amongst elements $X_{es}$ comprised in the updated population $S_{es}$, and comparing an objective function of $X_{optimal,es}$ with an objective function of the identified optimal element $X_{optimal}$ for updation of the identified optimal element $X_{optimal}$; (xiii) randomly replace variables of the updated population $S_{es}$ to obtain a perturbed set of population $S_P$; and (xiv) generate one or more predictive models based on the selected optimal subset of variables $X_{optimal}$.

In an embodiment, the one or more predefined criteria comprise: (i) subsets in which variables having inter correlation amongst each other below a first predefined threshold, (ii) subsets whose variables are correlated with the biological response Y above a second defined threshold or a system generated dynamic threshold; (iii) seeded subsets having variables whose sizes are less than a current subset size and (iv) a user defined preferences for the set of variables X.

In an embodiment, the at least one selected path $P_l$ is identified based on a relative error value associated with the variable $v_q$.

In an embodiment, the steps (ii) till (xiii) are iteratively performed either sequentially or in parallel across multiple processors threads.

In an embodiment, the steps (iv) till (vii) are iteratively performed until the one or more predefined criteria are met, and wherein the one or more predefined criteria further comprise at least one of (a) a predefined number of iterations, (b) frequency of the matrix subset of variables, (c) the optimal objective function reaching a predetermined value, and (d) an improvement in the objective function in subsequent iterations reaching a predefined saturation value or machine epsilon.

In an embodiment, the steps (ix) till (xii) are performed either sequentially or in parallel across multiple processors threads.

In an embodiment, the one or more hardware processors are further configured by the instructions to: generating a final optimal subset based on the identified optimal element $X_{optimal}$ for predicting biological responses of chemical compounds, upon satisfying the one or more predefined criteria, and upon iteratively performing the steps (ii) till (xiii).

In an embodiment, the step of initializing a population $S_{pop}$ comprising matrix subsets $\{X_1, X_2, \ldots X_{pop}\}$ of variables selected from the filtered set of variables $X_f$ is preceded by transforming the set of variables X to obtain a set of transformed variables; and filtering the set of transformed variables based on one or more statistical criteria and predefined thresholds to obtain a filtered set of variables $X_f$.

In yet another aspect, there are provided one or more non-transitory machine readable information storage mediums comprising one or more instructions which when executed by one or more hardware processors cause selecting one or more optimal variables from a set of unique variables and generating predictive models thereof by (i) receiving, via one or more hardware processors, a set of physico-chemical properties X derived from chemical structures of drugs and drug like chemical compounds, a biological response $Y$ associated thereof and a size of variables set r; (ii) initializing a population $S_{pop}$ comprising matrix subsets $\{X_1, X_2, ... X_{pop}\}$ of variables selected from a filtered set of variables $X_f$ of size $n$, wherein $X_f$ is derived from a set of variables X, wherein $S_{pop}$ is initialized based on one or more pre-defined criteria, wherein pop represents size of the population, and wherein each matrix subset $X_i$ from $S_{pop}$ comprises variables that are unique from each other and is of the size of variables set r; (iii) selecting at least one subset $X_i$ from $S_{pop}$ and at least one path $P_l$ of the at least one selected subset $X_i$, wherein the at least one selected path $P_l$ comprises a variable $v_q$ to be replaced, wherein each variable $v_q$ comprised in the at least one selected path $P_l$ is a vector of size m describing a property of input chemical compounds, and wherein m represents the number of chemical compounds used for building predictive models; (iv) replacing the variable $v_q$ from at least one subset Xiwith remaining $(n - r)$ variables of the set of variables $X_f$ to obtain a set of modified subsets $X_i' \{X_{i1}', X_{i2}', ..., X_{i(n-r)}'\}$, wherein each of the modified subsets $X_{ij}'$ comprises replaced variables for the at least selected path $P_l$, and wherein size of the set of modified subsets $X_i'$ is of $(n - r)$; (v) generating a predictive model for each of the modified subsets $X_{ij}'$, and calculating an objective function thereof to obtain a first set of predictive models $M_{i,rm}$ and associated objective functions $OF_{i,rm}$, wherein the first set of predictive models $M_{i,rm}$ are generated based on the biological response $Y$ and each of the modified subsets $X_{ij}'$ variable vectors of a set of input chemical compounds; (vi) identifying an optimal modified subset of replaced variables $X_i^{optimal}$ from the set of modified subsets $X_i'$ based on an optimal objective function associated with an optimal predictive model $M_i^{optimal}$ being identified from the first set of predictive models $M_{i,rm}$; (vii) updating the at least one selected path $P_l$ for the optimal modified subset of replaced variables $X_i^{optimal}$, wherein the steps (iv) till (vii) are iteratively performed until one or more predefined criteria are met to obtain an optimal population $S_{pop}^{optimal}$; (viii) identifying an optimal element $X_{optimal}$ of $S_{pop}^{optimal}$, wherein $X_{optimal}$ comprises an optimal objective function amongst objective functions comprised in other $X_i^{optimal}$; (ix) performing an exhaustive search on a pool of variable $X_{pool}$ created using the optimal population $S_{pop}^{optimal}$ to obtain a set of variable subsets $S_x$, wherein each element of $S_x$ comprises set of r variables; (x) generating the predictive model for each element of $S_x$ and calculating the objective function thereof to obtain a second set of predictive models $M_x$ and associated objective functions $OF_x$; (xi) identifying a $pop$ number of optimal elements from $S_x$ to update the optimal population $S_{pop}^{optimal}$ and to obtain an updated population $S_{es}$; (xii) identifying an optimal element $X_{optimal,es}$ amongst elements $X_{es}$ comprised in the updated population $S_{es}$, and comparing an objective function of $X_{optimal,es}$ with an objective function of the identified optimal element $X_{optimal}$ for updation of the identified optimal element $X_{optimal}$; (xiii) randomly replacing variables of the updated population $S_{es}$ to obtain a perturbed set of population $S_P$; and (xiv) generating one or more predictive models based on the selected optimal subset of variables $X_{optimal}$.

In an embodiment, the one or more predefined criteria comprise: (i) subsets in which variables having inter correlation amongst each other below a first predefined threshold, (ii) subsets whose variables are correlated with the biological response Y above a second defined threshold or a system generated dynamic threshold; (iii) seeded subsets having variables whose sizes are less than a current subset size and (iv) a user defined preferences for the set of variables X.

In an embodiment, the at least one selected path $P_l$ is identified based on a relative error value associated with the variable $v_q$.

In an embodiment, the steps (ii) till (xiii) are iteratively performed either sequentially or in parallel across multiple processors threads.

In an embodiment, the steps (iv) till (vii) are iteratively performed until the one or more predefined criteria are met, and wherein the one or more predefined criteria further comprise at least one of (a) a predefined number of iterations, (b) frequency of the matrix subset of variables, (c) the optimal objective function reaching a predetermined value, and (d) an improvement in the objective function in subsequent iterations reaching a predefined saturation value or machine epsilon.

In an embodiment, the steps (ix) till (xii) are performed either sequentially or in parallel across multiple processors threads.

The method may further comprise upon satisfying the one or more predefined criteria, and upon iteratively performing the steps (ii) till (xiii), generating a final optimal subset based on the identified optimal element $X_{optimal}$ for predicting biological responses of chemical compounds.

In an embodiment, the step of initializing a population $S_{pop}$ comprising matrix subsets $\{X_1, X_2, ... X_{pop}\}$ of variables selected from the filtered set of variables $X_f$ is preceded by transforming the set of variables $X$ to obtain a set of transformed variables; and filtering the set of transformed variables based on one or more statistical criteria and predefined thresholds to obtain a filtered set of variables $X_f$.

In an embodiment, the one or more predictive models comprise one or more linear regression models, one or more non-linear regression models, or one or more classification models, and wherein the one or more predictive models and the identified optimal subset $X_{optimal}$ are used for generating one or more rules or alerts.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for optimal variables selection for generating predictive models using population based exhaustive replacement techniques (or population based exhaustive replacement method (PERM)) in accordance with an embodiment of the present disclosure.

FIGS. 2A-2B illustrate an exemplary flow chart illustrating a method for optimal variables selection for generating predictive models using population based exhaustive replacement techniques implemented in the system 100 in accordance with an embodiment of the present disclosure.

FIGS. 3A-3B illustrate an exemplary flow diagram depicting a method for optimal variables selection for generating predictive models using population based exhaustive replacement techniques implemented in the system in accordance with an embodiment of the present disclosure.

FIG. 4A depicts a flow chart illustrating a method of performing replacement of variable $v_q$ from at least one subset $X_i$ for each selected path in a sequential manner as implemented by the system of FIG. 1 in accordance with an embodiment of the present disclosure.

FIG. 4B depicts a flow chart illustrating a method of performing replacement of variable $v_q$ from at least one subset $X_i$ for each selected path in parallel across multiple processors threads as implemented by the system of FIG. 1 in accordance with an embodiment of the present disclosure.

FIG. 5A depicts a flow chart illustrating a method of performing exhaustive replacement technique on variable subsets $S_x$, generating predictive model for each element of $S_x$, identifying a pop number of optimal elements from $S_x$ for obtaining an updated population $S_{es}$, and updation of the identified optimal element $X_{optimal}$ in a sequential manner as implemented by the system of FIG. 1 in accordance with an embodiment of the present disclosure.

FIG. 5B depicts a flow chart illustrating a method of performing exhaustive replacement technique on variable subsets $S_x$, generating predictive model for each element of $S_x$, identifying a pop number of optimal elements from $S_x$ for obtaining an updated population $S_{es}$, and updation of the identified optimal element $X_{optimal}$ in parallel across multiple processors threads as implemented by the system of FIG. 1 in accordance with an embodiment of the present disclosure.

FIG. 6 depict a flow chart illustrating a method of generating a perturbed set of population $S_P$, using the system of FIG. 1 in accordance with an embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

**GLOSSARY:**

Expression 'Subset' refers to a small set of elements which are taken from a larger set of elements/variables/features.

Expression 'Path' refers to a position of the element to be replaced in the subset.

Expression 'Descriptor(s)' refer to indices of the features/variables/attributes of a chemical compound.

Expression 'Exhaustive Search' refers to a technique of searching all the possible combinations of a given subset size.

Expression 'Replacement' refers to replacing a current descriptor with remaining descriptors.

Expression 'MLR - Multiple Linear Regression' refers to relationship between one continuous dependent variable and two or more independent variables.

Expression 'Objective Function' refers to a function that is desired to maximize or minimize any numeric value.

Expression 'Convergence' refers to an act of converging and especially moving toward union or uniformity.

Expression 'model' refers to a statistical predictive model having dependent variable and independent variables.

Expression 'Predictivity of Model' refers to an ability of the model to predict the biological responses of compound(s) using statistics. Biological response refers to the changes measured or predicted within a biological system e.g., drug targets, animals, humans on exposure to a drug, marketed or under development within a pharmaceutical industry.

Expression 'Correlation' refers to a statistical technique that is used to measure and describe the strength and direction of the relationship between two variables.

Expression 'Population' refers to a set of subsets having descriptors.

Expression 'R-Squared' refers to a coefficient of determination and is the proportion of the variance in the dependent variable that is predictable from by the independent variables.

Expression 'Optimal subset' refers to a subset which is having highest R-Squared in comparison with all the other possible subsets.

Expression 'Pool' refers to a group of descriptors which are highly correlated with the dependent variable.

Expression 'Deterministic' refers to no randomness and produces the same output from a given starting condition or initial state.

Expression 'Redundant' refers to data duplication.

Expression 'Over-fitting' refers to production of an analysis that corresponds too closely or exactly to a particular set of data and may therefore fail to fit additional data or predict future observations reliably.

Expression 'Standard-error/Standard-deviation' is a measure that is used to quantify the amount of variation or dispersion of a set of data values.

Expression 'Perturbation' refers to a method of introducing noise in the output or a subset.

All the above expressions shall be interpreted in light of the context and detailed description as described herein in the present disclosure.

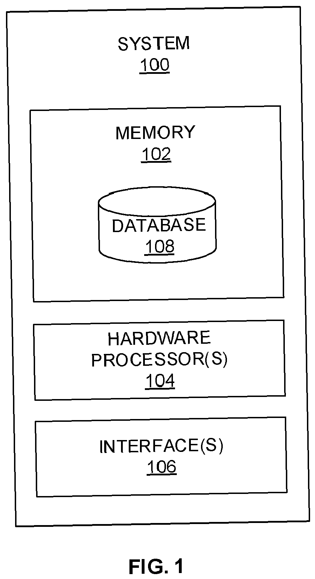

Referring now to the drawings, and more particularly to FIG. 1 through 6, where similar reference characters denote corresponding features consistently throughout the figures, there are shown preferred embodiments and these embodiments are described in the context of the following exemplary system and/or method.

FIG. 1 illustrates an exemplary block diagram of a system 100 for optimal variables selection for generating predictive models using population based exhaustive replacement techniques (or population based exhaustive replacement method (PERM)) in accordance with an embodiment of the present disclosure. The system 100 may also be referred as 'a PERM system' and interchangeably used hereinafter. In an embodiment, the system 100 includes one or more processors 104, communication interface device(s) or input/output (I/O) interface(s) 106, and one or more data storage devices or memory 102 operatively coupled to the one or more processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the device 100 can be implemented in a variety of computing systems, such as laptop computers, notebooks, hand-held devices, workstations, mainframe computers, servers, a network cloud and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment a database 108 is stored in the memory 102, wherein the database 108 may comprise, but are not limited to information pertaining to physico-chemical properties, chemical structures of drugs and drug like compounds, property of input chemical compounds, rules or alerts, various models that are generated and executed for prediction of biological response, predefined threshold values, configuration details of the system during training phase and test/validation phase to perform the methodology described herein. The memory 102 further comprises (or may further comprise) information pertaining to input(s)/output(s) of each step performed by the systems and methods of the present disclosure.

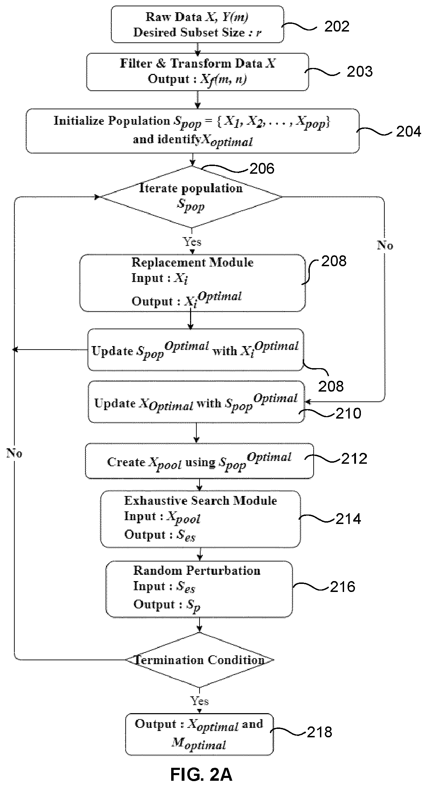

FIGS. 2A-2B, with reference to FIG. 1, illustrate an exemplary flow chart illustrating a method for optimal variables selection for generating predictive models using population based exhaustive replacement techniques implemented in the system 100 in accordance with an embodiment of the present disclosure.

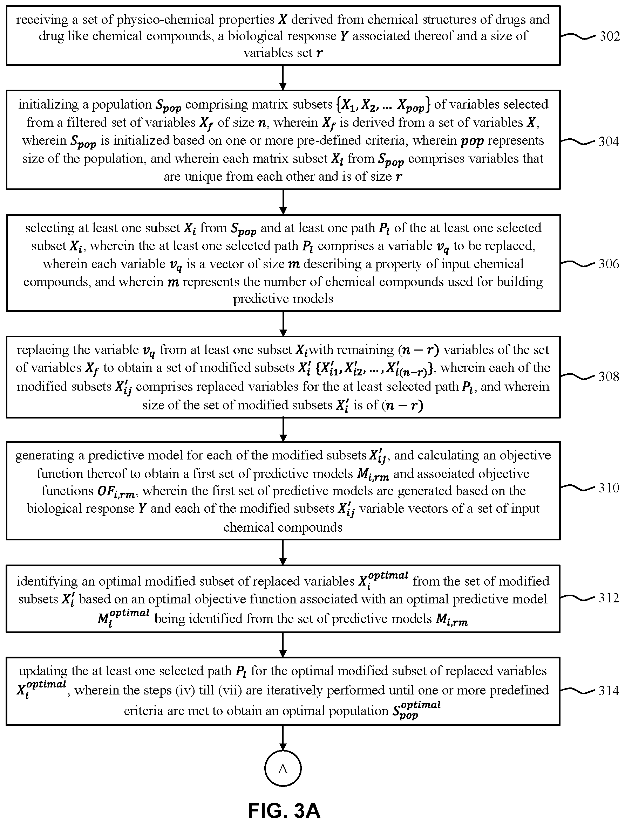

FIGS. 3A-3B, with reference to FIG. 1 through 2B, illustrate an exemplary flow diagram depicting a method for optimal variables selection for generating predictive models using population based exhaustive replacement techniques implemented in the system 100 in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, the flow charts FIGS. 2A-2B, FIGS. 4A-4B, FIGS. 5A-5B, FIG. 6 and the flow diagram as depicted in FIGS. 3A-3B. At step 302, the one or more hardware processors 104 receive, a set of physico-chemical properties X derived from chemical structures of drugs and drug like compounds, more generally chemical compounds, a biological response $Y$ associated thereof and a size of variables set r in the system 100 (e.g., refer step 202 of FIGS. 2A-2B). At step 304, the one or more hardware processors 104 initialize a population $S_{pop}$ comprising matrix subsets $\{X_1, X_2, ... X_{pop}\}$ of variables selected from a filtered set of variables $X_f$ of size $n$. $X_f$ is derived from a set of variables $X$, $S_{pop}$ is initialized based on one or more pre-defined criteria, pop represents size of the population and each matrix subset $X_i$ from $S_{pop}$ comprises variables that are unique from each other and is of the size of variables set r (e.g., refer 204 of FIG. 2A). Prior to initializing the population $S_{pop}$ comprising matrix subsets $\{X_1, X_2, ... X_{pop}\}$ of variables selected from the filtered set of variables $X_f$, the system 100 transforms the set of variables X to obtain a set of transformed variables and filters the set of transformed variables based on one or more statistical criteria and predefined thresholds to obtain a filtered set of variables $X_f$. The step of transformation and filtration are depicted in step 203 of FIG. 2A-2B.

In the present disclosure, the one or more predefined criteria comprise: (i) subsets in which variables have inter correlation amongst each other below a first predefined threshold, (ii) subsets whose variables are correlated with the biological response $Y$ above a second defined threshold or a system generated dynamic threshold; (iii) seeded subsets having variables whose sizes are less than a current subset size and (iv) a user defined preferences for the set of variables $X$.

At step 306, the one or more hardware processors 104 select at least one subset $X_i$ from $S_{pop}$ and at least one path $P_l$ of the at least one selected subset $X_i$, wherein the at least one selected path $P_l$ comprises a variable $v_q$ to be replaced. Each variable $v_q$ comprised in the at least one selected path $P_l$ is a vector of size m describing a property of input chemical compounds and wherein m represents the number of chemical compounds used for building predictive models. In the present disclosure, the at least one selected path $P_l$ is identified based on a relative error value associated with the variable $v_q$.

At step 308, the one or more hardware processors 104 replace the variable $v_q$ from at least one subset $X_i$ with remaining ($n$ - $r$) variables of the set of variables $X_f$ to obtain a set of modified subsets $X_i'\ \{X_{i1}', X_{i2}', ..., X_{i(n-r)}'\}$ , wherein each of the modified subsets $X_{ij}'$ comprises replaced variables for the at least selected path $P_l$, wherein size

of the set of modified subsets $X_i'$ is of $(n - r)$ (e.g., refer step 208 of FIG. 2A). At step 310, the one or more hardware processors generate a predictive model for each of the modified subsets $X_{ij}'$, and calculate an objective function thereof to obtain a first set of predictive models $M_{i,rm}$ and associated objective functions $OF_{i,rm}$. In the present disclosure, the first set of predictive models $M_{i,rm}$ is/are generated based on the biological response $Y$ and each of the modified subsets $X_{ij}'$ variable vectors of a set of input chemical compounds. At step 312, the one or more hardware processors 104 identify an optimal modified subset of replaced variables $X_i^{optimal}$ from the set of modified subsets $X_i'$ based on an optimal objective function associated with an optimal predictive model $M_i^{optimal}$ being identified from the first set of predictive models $M_{i,rm}$. At step 314, the one or more hardware processors 104 update the at least one selected path $P_i$ for the optimal modified subset of replaced variables $X_i^{optimal}$. The steps 308 till 312 are iteratively performed until one or more predefined criteria are met to obtain an optimal population $S_{pop}^{optimal}$ (e.g., refer step 210 of FIG. 2A). In the present disclosure, the one or more predefined criteria further comprise at least one of (a) a predefined number of iterations, (b) frequency of the matrix subset of variables, (c) the optimal objective function reaching a pre-determined value, and (d) an improvement in the objective function in subsequent iterations reaching a predefined saturation value or machine epsilon. In an embodiment, the one or more predefined criteria may also be referred as pre-determined criteria and interchangeably used herein.

At step 316, the one or more hardware processors 104 identify an optimal element $X_{optimal}$ of $S_{pop}^{optimal}$, wherein $X_{optimal}$ comprises an optimal objective function amongst objective functions comprised in other $X_i^{optimal}$.

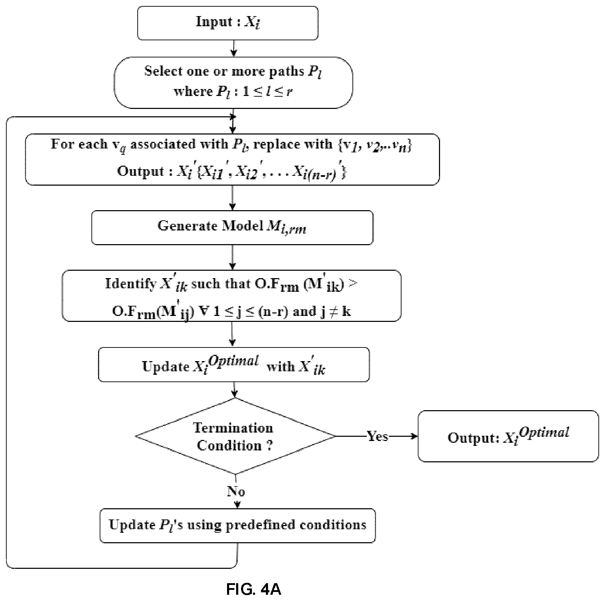

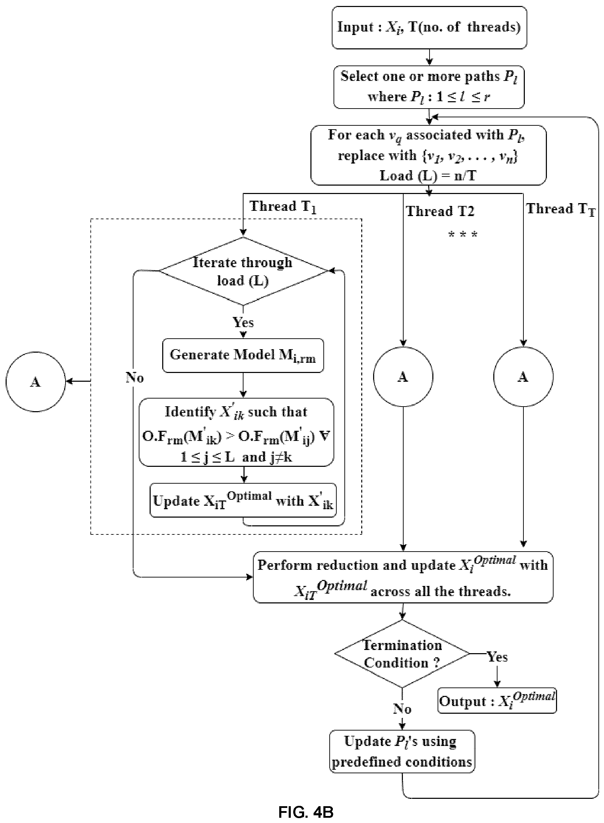

The steps 306 till 316 are described by way of a flow chart depicted in FIG. 4A and 4B in accordance with an embodiment of the present disclosure. More specifically, FIG. 4A, with reference to FIGS. 1-2, depicts a flow chart illustrating a method of performing replacement of variable $v_q$ from at least one subset $X_i$ for each selected path in a sequential manner as implemented by the system 100 in accordance with an embodiment of the present disclosure. FIG. 4B, with reference to FIGS. 1-2, depicts a flow chart illustrating a method of performing replacement of variable $v_i$ from at least one subset $X_i$ for each selected path in parallel across multiple processors threads as implemented by the system 100 in accordance with an embodiment of the present disclosure.

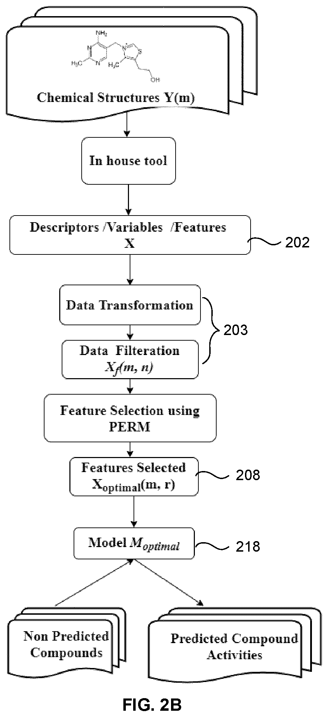

At step 318, the one or more hardware processors 104 perform an exhaustive search on a pool of variable $X_{pool}$ created (e.g., refer step 212 of FIG. 2) using the optimal population $S_{pop}^{optimal}$ to obtain a set of variable subsets $S_x$, wherein each element of $S_x$ comprises set of $r$ variables. At step 320, the one or more hardware processors 104 generate the predictive model for each element of $S_x$ and calculate the objective function thereof to obtain a second set of predictive models $M_x$ and associated objective functions $OF_x$. At step 322, the one or more hardware processors 104 identify a pop number of optimal elements from $S_x$ to update the optimal population $S_{pop}^{optimal}$ and to obtain an updated population $S_{es}$ (e.g., refer step 214 of FIG. 2). At step 324, the one or more hardware processors 104 identify an optimal element $X_{optimal,es}$ amongst elements $X_{es}$ comprised in the updated population $S_{es}$, and compare an objective function of $X_{optimal,es}$ with an objective function of the identified optimal element $X_{optimal}$ for updation of the identified optimal element $X_{optimal}$.

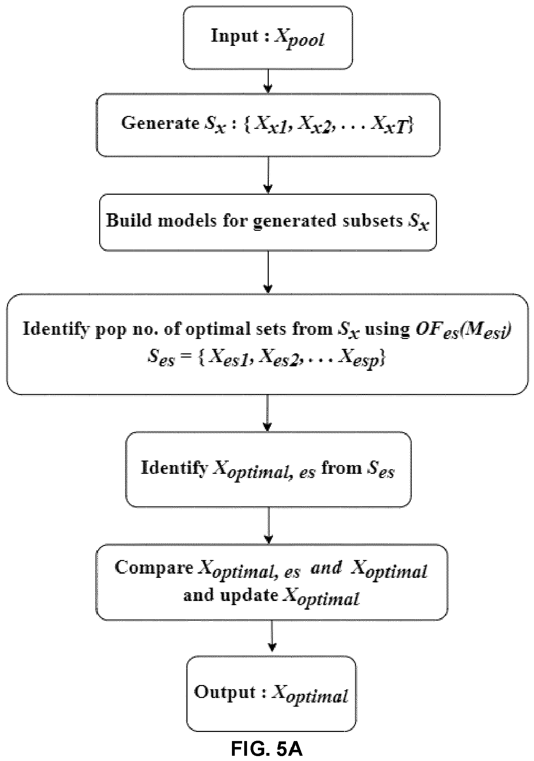

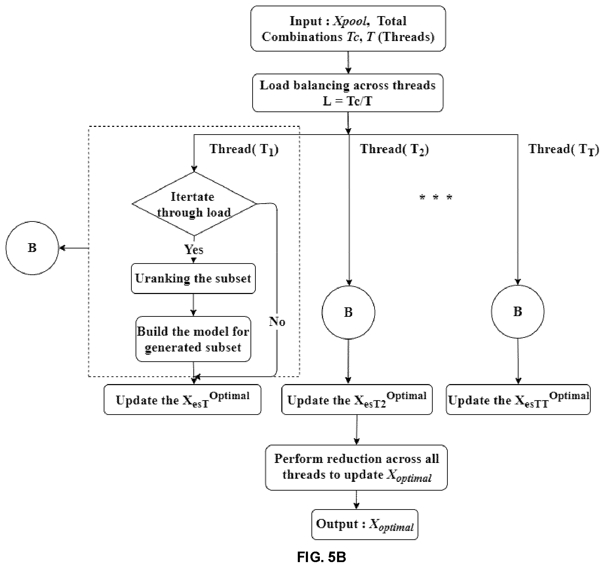

The steps 318 till 324 are described by way of a flow chart depicted in FIGS. 5A and 5B in accordance with an embodiment of the present disclosure. More specifically, FIG. 5A, with reference to FIGS. 1-4B, depicts a flow chart illustrating a method of performing exhaustive replacement technique (or exhaustive replacement method and may be interchangeably used herein) on variable subsets $S_x$, generating predictive model for each element of $S_x$, identifying a pop number of optimal elements from $S_x$ for obtaining an updated population $S_{es}$, and updation of the identified optimal element $X_{optimal}$ in a sequential manner as implemented by the system 100 in accordance with an embodiment of the present disclosure. FIG. 5B, with reference to FIGS. 1-5A, depicts a flow chart illustrating a method of performing exhaustive replacement technique on variable subsets $S_x$, generating predictive model for each element of $S_x$, identifying a *pop* number of optimal elements from $S_x$ for obtaining an updated population $S_{es}$, and updation of the identified optimal element $X_{optimal}$ in parallel across multiple processors threads as implemented by the system 100 in accordance with an embodiment of the present disclosure.

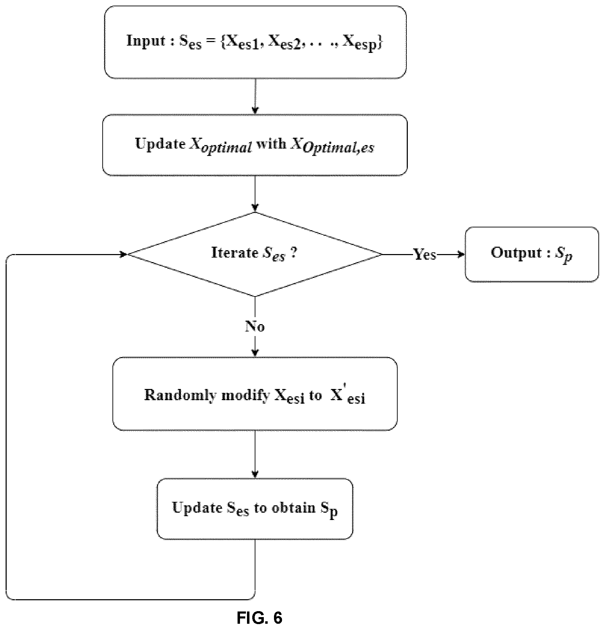

At step 326, the one or more hardware processors 104 randomly replace variables of the updated population $S_{es}$ to obtain a perturbed set of population $S_P$ (e.g., refer step 216 of FIG. 2). The step 326 is described by way of a flow chart depicted in FIG. 6 in accordance with an embodiment of the present disclosure. More specifically, FIG. 6, with reference to FIGS. 1 through 5B, depicts a flow chart illustrating a method of generating a perturbed set of population $S_P$, using the system 100 in accordance with an embodiment of the present disclosure. Upon satisfying the one or more predefined criteria, and upon iteratively performing the steps 304 till 326, the system 100 generates a final optimal subset based on the identified optimal element $X_{optimal}$ for predicting biological responses of chemical compounds.

In the present disclosure, the steps 304 till 326 are iteratively performed either sequentially or in parallel across multiple processors threads. Further, the steps 318 till 324 are performed either sequentially or in parallel across multiple processors threads for improved efficiency with respect to time associated with the parallelization of the population based exhaustive replacement method which provides technical solution to the technical problem of optimal variables/features selection by optimizing the processing time and eventually generates optimal predictive models.

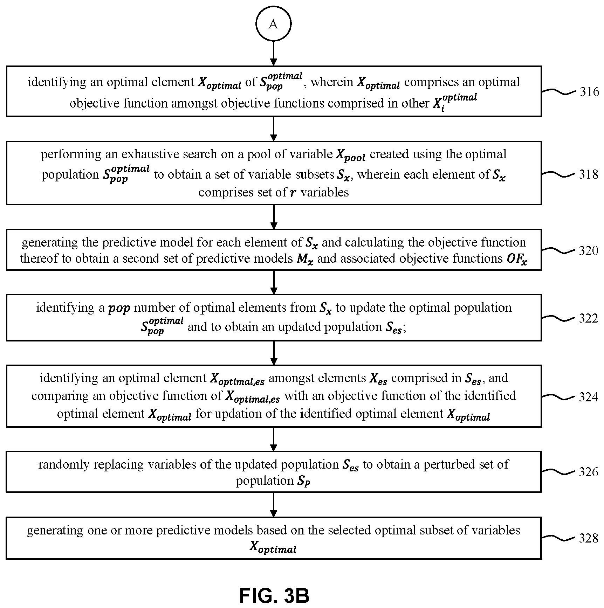

Referring back to FIG. 3B, at step 328, the one or more hardware processors 104 generate one or more predictive models based on the selected optimal subset of variables $X_{optimal}$. In the present disclosure, the one or more predictive models comprise, but are not limited to, one or more linear regression models, one or more non-linear regression models, or one or more classification models. The one or more predictive models and the identified optimal subset $X_{optimal}$ are used for generating one or more rules or alerts, in one example embodiment.

The modifications as discussed in the present disclosure in comparison to conventional approaches improve search capability of the method of the present disclosure and thus, larger number of variables can be selected, which improves the predictive performance generated models. Further, selected features provide insights with respect to the physico-chemical property or structural features to be optimized to improve the discovery of drug candidates with better property profiles, consequently, increasing the success, reduction in cost of drug discovery and development, within a pharmaceutical industry. The method of the present disclosure was also executed on various parallel architectures to demonstrate that Parallel PERM can be used to achieve better, and reliable results in real time even for larger datasets, wherein selection of larger variables is faster. Parallelization of PERM can be done on various shared and distributed memory architectures in which the workload is distributed among the threads to reduce computation memory and time. In an example embodiment, PERM can be parallelized (e.g., refer FIGS. 4B and 5B) for OpenMP and CUDA parallel implementations.

In an embodiment, when parallelizing PERM, the population initialization step can be executed in parallel on a number of compute threads that search for a subset with predefined conditions. As the population contains unique subsets, the parallel initialization of population can create some duplicates. The present disclosure may minimize the duplicate by using the population index as a seed to the random number generator, time, and others as appropriate.

In the same embodiment, the system can be parallelized in more than one number of ways. Some of which are described below:

i. Replacement for each of the $X_i$ of $S_{pop}$ can be executed on a separate thread i.e., *'pop'* number of threads can be executed simultaneously and independently for each subset

ii. Further, more than one threads, in parallel, can replace (*n - r*) descriptors in a selected path and build predictive models. The number of threads can range from a 0 to *(pop * 2 * (n - r))* for a single iteration of replacement technique. After which a reduction operation can be performed to obtain the optimal subset and the next paths to replace can be updated.

iii. The exhaustive search technique can be performed in parallel by distributing the subsets evenly among the threads. In this, each thread evaluates the combinations assigned to it one after the other and outputs $S_x^{thread}$ number of best subsets. After this a reduction operation can be performed on all of the threads $S_x^{thread}$ subsets to get $S_x$.

iv. Further, replacement and exhaustive search can also be executed on the same threads with intermediate reduction operations in parallel or replacement and reduction operation can be executed in serial and exhaustive search technique can be executed in parallel.

The present disclosure can be parallelized based on the architecture type and parallelization technology used. The replacement technique was parallelized on multi core using OpenMP and many core using CUDA.

In an example embodiment, the system was parallelized on OpenMP using above-described techniques and additional modifications as described below. In this, replacement (RM) was performed with T OpenMP threads on initial subset $X(m, r)$ that replaced in two different paths. For example, Path1 and Path2 were replaced with descriptors iteratively in the nested loops. In this the path2 replacement was executed in parallel using OpenMP. The load $(L = n/T)$ of replacing the Path2 was equally balanced across T threads. Each thread is having a range of ranks to the get the combination $X'(m, r)$ of descriptors, correlation checks, calculate objective function $O. F(X')$ and update $X_{Toptimal}$ with $X'$ if it is better. Each thread may have a thread optimal subset. Once all the threads evaluate the subsets assigned to them, objective functions across threads are compared and $X_{optimal}$ is updated with $X_{Toptimal}$.

In another example embodiment, the system executes the two nested loops of Path1 and Path2 in parallel simultaneously. In this parallel method, the number of combinations with replacing the two paths/positions with remaining descriptors were calculated. This load is balanced across T number of OpenMP threads. Each thread identifies the subsets it needs to evaluate and performs correlation check, objective function evaluation and updated $X_{Toptimal}$ with $X'$ if it is better. In this way each thread has a thread optimal subset. Once all the threads complete execution with their respective loads, O.F's across the threads are compared and $X_{optimal}$ is updated with $X_{Toptimal}$.

In yet another example embodiment, the present disclosure is executed in parallel on Nvidia graphics card using CUDA. In one example execution of sequential PERM $Path_1$ and $Path_2$ have been replaced with descriptors iteratively in the nested loops. These two nested loops were parallelized together in CUDA implementation and were executed on b blocks and t threads per block. Here the number of blocks and number of threads per block are equal to the number of descriptors n. For the given subset $X(m,r)$ $Path_1$ and $Path_2$ were replaced with the block index and thread index respectively. In this way each thread in the block has a modified subset X'(m, r) where it performs correlation check, calculates the objective function $O. F(X')$ and updated thread optimal subset. Subsequently, reduction operation was performed across the threads within the block and $X_{block,optimal}$ was calculated. In this way each block has $X_{Toptimal}$ with $Path_2$ replaced with best descriptor. Then the reduction operation is performed across the blocks and the $X_{optimal}$ is updated with $X_{block, optimal}$.

Below are experimental results conducted by the embodiments of the present disclosure for various case studies, relevant to drug discovery and development applications, with pharmaceutical industry.

**Case Study 1: Modeling in-vitro Human Serum Albumin (HSA) binding**

The binding affinity of new chemical entities (NCEs) to Human Serum Albumin (HSA) is one of the important ADMET properties considered in drug discovery and development. Probably, it is the most extensively studied protein because of its abundance, low cost, ease of purification and stability. It plays a central role in drug pharmacokinetics particularly in the distribution of drugs. Most drugs are transported in bound form to HSA and reach the target tissues. HSA allows solubilization of hydrophobic compounds, thus contributing to a homogeneous distribution of drugs in the body and increases their biological lifetime. Binding of a drug to serum albumin is a reversible process and is therefore in an equilibrium state. Only the unbound drug molecules contribute to the pharmacological efficacy; however, they are equally susceptible to metabolic reactions. Given the high concentration of albumin, the binding strength of any drug to serum albumin is the main factor that determines the availability of that drug and consequently, the diffusion of the drug from the circulatory system to target tissues. All these factors cause the pharmacokinetics of almost any drug to be dramatically influenced and controlled by its binding affinity to serum albumin.

In this case study, the system 100 built global QSPR models for HSA binding affinity using a dataset of 84 drugs and drug-like compounds, originally reported by Gonzalo Colmenarejo et al. keeping logK0hsa, as the dependent variable (Y). In the study by the embodiments of the present disclosure, a total of 392 physico-chemical descriptors were used in the QSPR model generation. These 392 molecular descriptors were calculated using the system of the present disclosure, and these fall into the following classes: (1) structural descriptors, (2) physico-chemical descriptors, (3) geometrical descriptors and (4) topological descriptors.

Herein, practical application of the present disclosure and its associated embodiments and systems and methods is discussed, and parallelization of the method of the present disclosure was performed to derive global QSPR models for human serum albumin binding affinity. In the case study as being discussed in the present disclosure, HSA has been modeled using multiple linear regression based on equation (1) given below and to evaluate the predictive ability of feature subsets built MLR model's correlation value ($r^2$) calculated was used using the equation (2).

$$\hat{y} = X\hat{\beta} = X(X'X)^{-1} X'y \qquad (1)$$

$$r^2 = \frac{\sum_i (y_{predicted,i} - y_{observed,i}))^2}{\sum_i (y_{observed,i} - y_{mean}))^2} \qquad (2)$$

The present disclosure has transformed the generated 396 descriptors into various forms such as square, exponential, logarithmic and others and filtered each transformation using a correlation threshold with Y of above 0.1. The system 100 has filtered 288 out of 396 descriptors using this criterion. Reproducibility of the present disclosure was compared by executing the system for ten runs, consistency of results across these runs were observed. It is observed that PERM is able to generate reproducible selection of variables and regressions models and thus, is an improved solution, thereby increased the reliability of the model results. Table 1 below compares the results of PERM with conventional approaches (ERM).

Table 1

| Subset Size | ERM | | | PERM | | |
|---|---|---|---|---|---|---|
| | Optimal Subset | Optimal $r^2$ | Reproducibility | Optimal Subset | Optimal $r^2$ | Reproducibility |
| 5 | 102, 165, 250, 269, 273 | 0.842 | 10/10 | 102, 165, 250, 269, 273 | 0.842 | 10/10 |
| 6 | 102, 104, 165, 250, 269, 273 | 0.857 | 9/10 | 102, 104, 165, 250, 269, 273 | 0.857 | 10/10 |
| 7 | 89, 102, 165, 243, 250, 269, 273 | 0.871 | 9/10 | 89, 102, 165, 243, 250, 269, 273 | 0.871 | 10/10 |
| 8 | 102, 120, 145, 165, 243, 250, 269, 273 | 0.884 | 5/10 | 102, 120, 145, 165, 243, 250, 269, 273 | 0.884 | 10/10 |
| 9 | 36, 102, 112, 165, 197, 243, 250, 269, 273 | 0.891 | 1/10 | 36, 102, 112, 165, 197, 243, 250, 269, 273 | 0.891 | 10/10 |

For the above case study, a set of two to three subsets with inter correlation values $r^2_{x_i,x_j}$ greater than median value of inter correlation and one to three subsets with $r_{xy,thres}$ greater than mean correlation values of dependent variables with Y were taken as initial subsets in PERM (population based exhaustive replacement method of the present disclosure). The stopping criteria for PERM was to execute one to two iterations of replacement and exhaustive methods one after another. In addition, each replacement technique internally executed for two to four iterations.

Further, PERM of the present disclosure was parallelized on two high performance computer architectures 1. Open multi-processing (OpenMP) and 2. Compute unified device architecture (CUDA) on a graphical processing unit. The configuration of the system used in this case study is Intel(R) Xeon (R) CPU E5-2609 v3 @ 1.90GHz with 6 cores with NVIDIA Quadro K4200 (GPU). Below Table 2 depicts comparison of Serial and Parallel PERM approaches as described by the present disclosure herein.

Table 2

| Data | Subset Size | Architecture | Number of Threads | Optimal Subset | Optimal $r^2$ | Time (sec) | Speed Up (~) |
|---|---|---|---|---|---|---|---|
| 84 Compounds X 288 Descriptors | 5 | Serial | 1 | 102, 165, 250, 269, 273 | 0.841 | 20 | 1 |
| | | OpenMP | 6 | 102, 165, 250, 269, 273 | 0.841 | 4 | 5 |
| | | CUDA | (288 blocks X 288 threads) | 102, 165, 250, 269, 273 | 0.841 | 1 | 20 |
| | 6 | Serial | 1 | 102, 104, 165, 250, 269, 273 | 0.857 | 37 | 1 |
| | | OpenMP | 6 | 102, 104, 165, 250, 269, 273 | 0.857 | 3 | 12.6 |
| | | CUDA | (288 blocks X 288 threads) | 102, 104, 165, 250, 269, 273 | 0.857 | 2 | 18.5 |

| | | | | | | | |
|---|---|---|---|---|---|---|---|
| | 7 | Serial | 1 | 89, 102, 165, 243, 250, 269, 273 | 0.871 | 45 | 1 |
| | | OpenMP | 6 | 89, 102, 165, 243, 250, 269, 273 | 0.871 | 3 | 15 |
| | | CUDA | (288 blocks X 288 threads) | 89, 102,165, 243, 250, 269, 273 | 0.871 | 3 | 15 |
| | 8 | Serial | 1 | 102, 120, 145, 165, 243, 250, 269, 273 | 0.884 | 76 | 1 |
| | | OpenMP | 6 | 102, 120, 145, 165, 243, 250, 269, 273 | 0.884 | 16 | 4.8 |
| | | CUDA | (288 blocks X 288 threads) | 102, 120, 145, 165, 243, 250, 269, 273 | 0.884 | 6 | 12.7 |
| | 9 | Serial | 1 | 86, 102, 104, 120, 165, 232, 250, 269, 273 | 0.889 | 106 | 1 |
| | | OpenMP | 6 | 102, 112, 165, 197, 199, 243, 250, 269, 273 | 0.891 | 24 | 4.4 |
| | | CUDA | (288 blocks | 6, 102, 146, 165, 197, | 0.890 | 11 | 9.6 |

| | | X 288 threads) | 231, 250, 269, 273 | | | |
|---|---|---|---|---|---|---|
| | Serial | 1 | 6, 105, 166, 197, 221, 224, 243, 250, 269, 274 | 0.902 | 228 | 1 |
| 10 | OpenMP | 6 | 36, 102, 117, 120, 145, 165, 243, 250, 269, 273 | 0.897 | 33 | 7 |
| | CUDA | (288 blocks X 288 threads) | 36, 54, 105, 153, 198, 216, 250, 269, 274, 284 | 0.898 | 15 | 15 |

The serial, OpenMP and CUDA versions of parallel PERM have been written in C language, in one example embodiment of the present disclosure. In addition, Ofast or O3 gcc compiler optimization were used while compiling serial and OpenMP codes of the algorithm. Speed up of 5-15x was achieved using OpenMP and 9-20x using CUDA for various subset sizes.

Below Table 3 depicts selected Optimal Descriptors for predicting the human serum albumin binding affinity of drugs and drug like compounds.

Table 3

| Descriptor Number | Name ($x$) | Transformation |
|---|---|---|
| 36 | Eccentric adjacency index | $x^3$ |
| 89 | Kier & Hall valence connectivity index order 5 | $\log x$ |
| 102 | Delta connectivity index Order 3(cluster) | $1/x$ |
| 104 | Delta connectivity index Order 4(cluster) | $x^3$ |
| 112 | Charge transfer index - algebraic semisum | $1/x$ |
| 117 | Charge index(G) Order 4 | $1/x$ |
| 120 | Valence charge index (Gv) Order 1 | $1/x$ |
| 145 | Autocorrelation descriptor (Broto-Moreau) weighted by atomic masses Order 0 | $1/x$ |
| 165 | Autocorrelation descriptor (Broto-Moreau) weighted by van der Waals radius Order 2 | $e^x$ |
| 197 | Autocorrelation descriptor (Geary) weighted by atomic van der Waals radius Order 1 | $x^3$ |
| 243 | Hydrogen Electro-topological state index (E-state): SHCsatu | $x$ |
| 250 | Atomic-Level-Based AI topological descriptors: AIdsCH | $e^x$ |
| 269 | AlogP98 (calculated logP) | $x$ |
| 273 | SKlogS in buffer system: logS | $x$ |

**Case Study 2: Modeling Epidermal growth factor receptor (EGFR)**

In another example embodiment, the present disclosure is used to predict Epidermal growth factor receptor (EGFR) kinase binding affinity of drugs and drug candidates. EGFR Kinase a transmembrane glycoprotein, is a proven anticancer target and many EGFR inhibitors such as Gefitinib, lapatinib, etc. have been developed and approved by the FDA for the treatment of breast cancer. In this case study, the applications of the present disclosure for the prediction of potential EGFR kinase inhibitors with high binding affinity is demonstrated and thus, the impact of the solution for new discovery within drug discovery and development.

In this case study, the dataset was obtained from publicly available kinase knowledge base. A number of filters were applied on the 93750 data entries obtain from public to maintain homogeneity of data. Out of these 4892 compounds were selected with in-vitro EGFR kinase enzyme binding assay pIC50 data for demonstrating the application of the technical solution described in the present disclosure. The following data processing steps were performed to select these compounds.

a. From the data, entries containing pIC50 for Enzyme assay "EGFR" type, were filtered resulting in 13940 entries.
b. Out of these, only the entries that have exact value of pIC50 were selected, i.e., entries whose relation with pIC50 is "=" relation for pIC50.
c. The 7255 entries resulted from above operation contained information on 5278 unique (MR_ID) compounds which contain one or more entries of pIC50 measurements. For compounds containing more than one value of pIC50, average of all readings was computed and used for modeling.
d. Finally, salt, or inorganic compounds were removed, if any, for further modeling.

The system 100, after filtering and computing the values of pIC50, computed 352 physico-chemical descriptors for the selected 4892 compounds that fall into the following classes: (1) structural descriptors, (2) physico-chemical descriptors, (3) geometrical descriptors and (4) topological descriptors. These 352 descriptors were filtered using zero variance, resulting in 350 descriptors for modelling.

The pIC50 values of these 4893 selected compounds ranges from 2.18-11.6. Based on pIC50 values, a stratified random sampling of the dataset was performed in ratio of 70:30 to create training and test dataset, resulting in train data with 3430 compounds and test data with 1463 compounds.

Herein, practical application of the present disclosure and its associated embodiments and systems and methods is discussed, and parallelization of the method of the present disclosure was performed to derive global QSPR models for EGFR binding. Like the previous case study discussed herein, the current case study models EGFR binding using multiple linear regression expression in equation (1) given below and to evaluate the predictive ability of feature subsets built MLR model's correlation value ($r^2$) calculated was used based on the equation (2).

$$\hat{y} = X\hat{\beta} = X(X'X)^{-1} X'y \qquad (1)$$

$$r^2 = \frac{\sum_i (y_{predicted,i} - y_{observed,i}))^2}{\sum_i (y_{observed,i} - y_{mean}))^2} \qquad (2)$$

Similarly, in this case study a subset with inter correlation values $r^2_{x_i,x_j}$ greater than median of inter correlation values and one subset with $r_{xy,thres}$ greater than mean correlation values of dependent variables with Y were taken as initial subsets in PERM. The stopping criteria for PERM was to execute two iterations of replacement and exhaustive methods one after another.

In this example embodiment, the system 100 has executed PERM in parallel on OpenMP and CUDA. The configuration of the system used in this case study has been Intel Xeon E5-2620 v2 @ 2.1 GHz with 24 cores machine and with Tesla K20 GPU. Table 4 depicts comparison of Parallel PERM on OpenMP and CUDA architectures.

Table 4

| Data | Subset Size | Architecture | Number of Threads | Optimal Subset | Optimal $r^2$ | Time (sec) | Speed Up (~) |
|---|---|---|---|---|---|---|---|
| 3430 Compounds X 350 Descriptors | 7 | OpenMP | 24 | 49, 200, 201, 221, 229, 247, 305 | 0.4065 | 176 | 1 |
| | | CUDA | (350 blocks X 350 threads) | 49, 200, 201, 221, 229, 247, 305 | 0.4065 | 80 | 2.2 |

| | | | | | | |
|---|---|---|---|---|---|---|
| 8 | OpenMP | 24 | 49, 200, 201, 221, 229, 247, 305, 314 | 0.420 | 512 | 1 |
| | CUDA | (350 blocks X 350 threads) | 49, 200, 201, 221, 229, 247, 305, 314 | 0.420 | 117 | 4.3 7 |
| 9 | OpenMP | 24 | 49, 166, 200, 201, 205, 221, 229, 305, 314 | 0.4329 | 783 | 1 |
| | CUDA | (350 blocks X 350 threads) | 49, 112, 166, 200, 201, 221, 229, 247, 305 | 0.4301 | 209 | 3.8 |
| 10 | OpenMP | 24 | 49,112,163, 200, 201, 221, 230, 241, 247, 305 | 0.4409 | 1319 | 1 |
| | CUDA | (350 blocks X 350 threads) | 49,112,163, 200, 201, 221, 230, 241, 247, 305 | 0.4409 | 290 | 4.5 4 |
| 15 | OpenMP | 24 | 49, 111, 161, 163, 166, 200, 201, 221, 228, | 0.4771 | 3250 | 1 |

| | | | | | | |
|---|---|---|---|---|---|---|
| | | | | 241, 305, 314, 318, 335, 349 | | | |
| | | CUDA | (350 blocks X 350 threads) | 49, 62,112,164,178,187,189,191, 201, 221, 229, 241, 242, 247, 305 | 0.4727 | 1722 | 1.88 |
| | 20 | OpenMP | 24 | 12, 37, 46, 49, 54, 58, 121, 157, 161, 166,199, 200, 201, 212, 220, 241, 305, 314, 320, 349 | 0.5008 | 293612 | 1 |
| | | CUDA | (350 blocks X 350 threads) | 44, 46, 49, 58, 65, 66, 95,121,186,187,191, 197, 201, 213, 230, 247, 254, 305, 340, 349 | 0.5029 | 52707 | 5.57 |

The OpenMP and CUDA versions of parallel PERM have been written in C language. In addition, O3 gcc compiler optimization was used while compiling OpenMP code of the algorithm. Speed up of 1.88x to 5.57x was achieved with CUDA in comparison to OpenMP parallel version with various subset sizes.

The present disclosure has also validated the selected feature/variable subsets on the test set compounds. Below Table 5 gives the results for test set prediction (e.g., results of PERM on Test Set)

| Table 5Subset Size | Optimal Subset | Train $r^2$ | Test $r^2$ |
|---|---|---|---|
| 7 | 49, 200, 201, 221, 229, 247, 305 | 0.4065 | 0.3944 |
| 8 | 49, 200, 201, 221, 229, 247, 305, 314 | 0.420 | 0.4088 |
| 9 | 49, 112, 166, 200, 201, 221, 229, 247, 305 | 0.4301 | 0.4091 |
| 10 | 49,112,163, 200, 201, 221, 230, 241, 247, 305 | 0.4409 | 0.4167 |
| 15 | 49, 62,112,164,178,187,189,191, 201, 221, 229, 241, 242, 247, 305 | 0.4727 | 0.4531 |
| 20 | 44, 46, 49, 58, 65, 66, 95,121,186,187,191, 197, 201, 213, 230, 247, 254, 305, 340, 349 | 0.5029 | 0.4835 |

While the drawbacks of conventional approaches as described above are imminent, these are very critical when applied to domain such as Pharma and thus very essential to address them effectively. It is also implicit that tuning the control parameters, increases computational time. Embodiments of the present disclosure have shown in results how performing some critical operations such as good initial point selection, exhaustive search are essential to achieve reliable results rather than mere tuning parameters. These modifications as suggested by the present disclosure are not a logical extension of conventional approaches, except for replacing multiple positions simultaneously, rather are essential elements to achieve quality results for any feature/variable selection methods and the solution provided in the current invention, is demonstrated to have the ability to select large number of variables (e.g., twenty). Selecting large number of variables for regression model generation has great impact in solving real world predictive regressions problems within the pharmaceutical industry. The selected variables offer key insights to practicing scientists involved in optimizing drug candidates and hence, the solution described in the present disclosure by system and method can improve the overall productivity, in terms of cost and time of drug discovery.

Embodiments of the present disclosure overcome the technical problems of selecting the best possible features from an extremely large number of features in real time with better predictive models, faster convergence, and reduced complexity of the models. The above technical problems have been overcome by the present disclosure by (i) use of transformed data for building regression models that ensures that any non-linear dependencies are also considered in model building, (ii) selecting initial population with a set of pre-defined criteria ensures a better initial point selection and helps in better convergence rate of the solution, (iii) multiple initial points rather than a single initial point on which further replacements are performed minimizes the chances of local optimization and improves the search capability of the model, (iv) exhaustive search ensures that the best model rather than optimal is derived out of the acquired knowledge - which improves predictability and can potentially improve reproducibility of the model, (v) replacing more than one descriptor in an iteration increases reproducibility and faster convergence as it minimizes the error due to assumption that the model is poor due to effect of a single variable, (vi) random perturbations in exhaustive search minimize the chances of local optimizations, (vii) provisioning parallelization of the PERM as implemented by the system 100 of the present disclosure on various architectures to achieve results in real time with compute time decreased by a factor of 20.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise. Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented population based exhaustive replacement method for selecting one or more optimal variables from a set of unique variables and generating predictive models thereof, comprising:

   (i) receiving, via one or more hardware processors, a set of physico-chemical properties X derived from chemical structures of drugs and drug like chemical compounds, a biological response $Y$ associated thereof and a size of variables set r (302);

   (ii) initializing, via the one or more hardware processors, a population $S_{pop}$ further comprising matrix subsets $\{X_1, X_2, ... X_{pop}\}$ of variables selected from a filtered set of variables $X_f$ of size $n$, wherein $X_f$ is derived from a set of variables $X$, wherein $S_{pop}$ is initialized based on one or more pre-defined criteria, wherein pop represents size of the population, and wherein each matrix subset $X_i$ from $S_{pop}$ comprises variables that are unique from each other and is of the size of variables set $r$ (304);

   (iii) selecting, via the one or more hardware processors, at least one subset $X_i$ from $S_{pop}$ and at least one path $P_l$ of the at least one selected subset $X_i$, wherein the at least one selected path $P_l$ comprises a variable $v_q$ to be replaced, wherein each variable $v_q$ comprised in the at least one selected path $P_l$ is a vector of size $m$ describing a property of input chemical compounds, and wherein $m$ represents a number of chemical compounds used for building predictive models (306);

   (iv) replacing, via the one or more hardware processors, the variable $v_q$ from at least one subset Xiwith remaining $(n - r)$ variables of the set of variables $X_f$ to obtain a set of modified subsets $X'_i \{X'_{i1}, X'_{i2}, ..., X'_{i(n-r)}\}$ , wherein each of the modified subsets $X'_{ij}$ comprises replaced variables for the at least selected path $P_l$, and wherein size of the set of modified subsets $X'_i$ is of $(n - r)$ (308);

   (v) generating, via the one or more hardware processors, a predictive model for each of the modified subsets $X'_{ij}$ , and calculating an objective function thereof to obtain a first set of predictive models $M_{i,rm}$ and associated objective functions $OF_{i,rm}$, wherein the first set of predictive models $M_{i,rm}$ are generated based on the biological response $Y$ and each of the modified subsets $X'_{ij}$ variable vectors of a set of input chemical compounds (310);

   (vi) identifying, via the one or more hardware processors, an optimal modified subset of replaced variables $X_i^{optimal}$ from the set of modified subsets $X'_i$ based on an optimal objective function associated with an optimal predictive model $M_i^{optimal}$ being identified from the first set of predictive models $M_{i,rm}$ (312);

   (vii) updating, via the one or more hardware processors, the at least one selected path $P_l$ for the optimal modified subset of replaced variables $X_i^{optimal}$ , wherein the steps (iv) till (vii) are iteratively performed until one or more predefined criteria are met to obtain an optimal population $S_{pop}^{optimal}$ (314);

   (viii) identifying, via the one or more hardware processors, an optimal element $X_{optimal}$ of $S_{pop}^{optimal}$ , wherein $X_{optimal}$ comprises an optimal objective function amongst objective functions comprised in other $X_i^{optimal}$ (316);

(ix) performing, via the one or more hardware processors, an exhaustive search on a pool of variable $X_{pool}$ created using the optimal population $S_{pop}^{optimal}$ to obtain a set of variable subsets $S_x$, wherein each element of $S_x$ comprises set of $r$ variables (318);

(x) generating, via the one or more hardware processors, the predictive model for each element of $S_x$ and calculating the objective function thereof to obtain a second set of predictive models $M_x$ and associated objective functions $OF_x$ (320);

(xi) identifying, via the one or more hardware processors, a $pop$ number of optimal elements from $S_x$ to update the optimal population $S_{pop}^{optimal}$ and to obtain an updated population $S_{es}$ (322);

(xii) identifying, via the one or more hardware processors, an optimal element $X_{optimal,es}$ amongst elements $X_{es}$ comprised in the updated population $S_{es}$, and comparing an objective function of $X_{optimal,es}$ with an objective function of the identified optimal element $X_{optimal}$ for updation of the identified optimal element $X_{optimal}$ (324);

(xiii) randomly replacing, via the one or more hardware processors, variables of the updated population $S_{es}$ to obtain a perturbed set of population $S_P$ (326); and

(xiv) generating one or more predictive models based on the selected optimal subset of variables $X_{optimal}$ (328).

2. The processor implemented population based exhaustive replacement method of claim 1, wherein the one or more predefined criteria comprise: (i) subsets in which variables having inter correlation amongst each other below a first predefined threshold, (ii) subsets whose variables are correlated with the biological response Y above a second defined threshold or a system generated dynamic threshold; (iii) seeded subsets having variables whose sizes are less than a current subset size and (iv) a user defined preferences for the set of variables X, wherein the at least one selected path $P_l$ is identified based on a relative error value associated with the variable $v_q$, and wherein the steps (ii) till (xiii) are iteratively performed either sequentially or in parallel across multiple processors threads.

3. The processor implemented population based exhaustive replacement method of claim 1, wherein the steps (iv) till (vii) are iteratively performed until the one or more predefined criteria are met, wherein the one or more predefined criteria further comprise at least one of (a) a predefined number of iterations, (b) frequency of the matrix subset of variables, (c) the optimal objective function reaching a predetermined value, and (d) an improvement in the objective function in subsequent iterations reaching a predefined saturation value or machine epsilon, and wherein the steps (ix) till (xii) are performed either sequentially or in parallel across multiple processors threads.

4. The processor implemented population based exhaustive replacement method of claim 2, further comprising upon satisfying the one or more predefined criteria, and upon iteratively performing the steps (ii) till (xiii), generating a final optimal subset based on the identified optimal element $X_{optimal}$ for predicting biological responses of chemical compounds.

5. The processor implemented population based exhaustive replacement method of claim 1, wherein the step of initializing a population $S_{pop}$ further comprising matrix subsets $\{X_1, X_2, ... X_{pop}\}$ of variables selected from the filtered set of variables $X_f$ is preceded by transforming the set of variables X to obtain a set of transformed variables; and filtering the set of transformed variables based on one or more statistical criteria and predefined thresholds to obtain a filtered set of variables $X_f$, wherein the one or more predictive models comprise one or more linear regression models, one or more non-linear regression models, or one or more classification models, and wherein the one or more predictive models and the identified optimal subset $X_{optimal}$ are used for generating one or more rules or alerts.

6. A processor implemented population based exhaustive replacement system (100) for selecting one or more optimal variables from a set of unique variables and generating predictive models thereof, comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

(i) receive, a set of physico-chemical properties X derived from chemical structures of drugs and drug like chemical compounds, a biological response Y associated thereof and a size of variables set $r$;
(ii) initialize a population $S_{pop}$ further comprising matrix subsets $\{X_1, X_2, ... X_{pop}\}$ of variables selected from a filtered set of variables $X_f$ of size $n$, wherein $X_f$ is derived from a set of variables $X$, wherein $S_{pop}$ is initialized based on one or more pre-defined criteria, wherein pop represents size of the population, and wherein each matrix subset $X_i$ from $S_{pop}$ comprises variables that are unique from each other and is of the size of variables set $r$;

(iii) select at least one subset $X_i$ from $S_{pop}$ and at least one path $P_l$ of the at least one selected subset $X_i$, wherein the at least one selected path $P_l$ comprises a variable $v_q$ to be replaced, wherein each variable $v_q$ comprised in the at least one selected path $P_l$ is a vector of size $m$ describing a property of input chemical compounds, and wherein $m$ represents a number of chemical compounds used for building predictive models;

(iv) replace the variable $v_q$ from at least one subset $X_i$ with remaining $(n - r)$ variables of the set of variables $X_f$ to obtain a set of modified subsets $X_i' \{X_{i1}', X_{i2}', \dots, X_{i(n-r)}'\}$, wherein each of the modified subsets $X_{ij}'$ comprises replaced variables for the at least selected path $P_l$, and wherein size of the set of modified subsets $X_i'$ is of $(n - r)$;

(v) generate a predictive model for each of the modified subsets $X_{ij}'$, and calculating an objective function thereof to obtain a first set of predictive models $M_{i,rm}$ and associated objective functions $OF_{i,rm}$, wherein the first set of predictive models $M_{i,rm}$ are generated based on the biological response $Y$ and each of the modified subsets $X_{ij}'$ variable vectors of a set of input chemical compounds;

(vi) identify an optimal modified subset of replaced variables $X_i^{optimal}$ from the set of modified subsets $X_i'$ based on an optimal objective function associated with an optimal predictive model $M_i^{optimal}$ being identified from the first set of predictive models $M_{i,rm}$;

(vii) update the at least one selected path $P_l$ for the optimal modified subset of replaced variables $X_i^{optimal}$, wherein the steps (iv) till (vii) are iteratively performed until one or more predefined criteria are met to obtain an optimal population $S_{pop}^{optimal}$;

(viii) identify an optimal element $X_{optimal}$ of $S_{pop}^{optimal}$, wherein $X_{optimal}$ comprises an optimal objective function amongst objective functions comprised in other $X_i^{optimal}$;

(ix) perform an exhaustive search on a pool of variable $X_{pool}$ created using the optimal population $S_{pop}^{optimal}$ to obtain a set of variable subsets $S_x$, wherein each element of $S_x$ comprises set of $r$ variables;

(x) generate the predictive model for each element of $S_x$ and calculating the objective function thereof to obtain a second set of predictive models $M_x$ and associated objective functions $OF_x$;

(xi) identify a *pop* number of optimal elements from $S_x$ to update the optimal population $S_{pop}^{optimal}$ and to obtain an updated population $S_{es}$;

(xii) identify an optimal element $X_{optimal,es}$ amongst elements $X_{es}$ comprised in the updated population $S_{es}$, and comparing an objective function of $X_{optimal,es}$ with an objective function of the identified optimal element $X_{optimal}$ for updation of the identified optimal element $X_{optimal}$;

(xiii) randomly replace variables of the updated population $S_{es}$ to obtain a perturbed set of population $S_P$; and

(xiv) generate one or more predictive models based on the selected optimal subset of variables $X_{optimal}$.

7. The processor implemented population based exhaustive replacement system of claim 6, wherein the one or more predefined criteria comprise: (i) subsets in which variables having inter correlation amongst each other below a first predefined threshold, (ii) subsets whose variables are correlated with the biological response $Y$ above a second defined threshold or a system generated dynamic threshold; (iii) seeded subsets having variables whose sizes are less than a current subset size and (iv) a user defined preferences for the set of variables $X$, wherein the at least one selected path $P_l$ is identified based on a relative error value associated with the variable $v_q$, and wherein the steps (ii) till (xiii) are iteratively performed either sequentially or in parallel across multiple processors threads.

8. The processor implemented population based exhaustive replacement system of claim 6, wherein the steps (iv) till (vii) are iteratively performed until the one or more predefined criteria are met, wherein the one or more predefined criteria further comprise at least one of (a) a predefined number of iterations, (b) frequency of the matrix subset of variables, (c) the optimal objective function reaching a predetermined value, and (d) an improvement in the objective function in subsequent iterations reaching a predefined saturation value or machine epsilon, and wherein the steps (ix) till (xii) are performed either sequentially or in parallel across multiple processors threads.

9. The processor implemented population based exhaustive replacement system of claim 7, wherein the one or more hardware processors are further configured by instructions to: upon satisfying the one or more predefined criteria, and upon iteratively performing the steps (ii) till (xiii), generate a final optimal subset based on the identified optimal element $X_{optimal}$ for predicting biological responses of chemical compounds.

10. The processor implemented population based exhaustive replacement system of claim 6, wherein the step of initializing a population $S_{pop}$ further comprising matrix subsets $\{X_1, X_2, ... X_{pop}\}$ of variables selected from the filtered set of variables $X_f$ is preceded by transforming the set of variables $X$ to obtain a set of transformed variables; and filtering the set of transformed variables based on one or more statistical criteria and predefined thresholds to obtain a filtered set of variables $X_f$, wherein the one or more predictive models comprise one or more linear regression models, one or more non-linear regression models, or one or more classification models, wherein the one or more predictive models and the identified optimal subset $X_{optimal}$ are used for generating one or more rules or alerts.

11. One or more non-transitory machine-readable information storage mediums comprising one or more instructions which when executed by one or more hardware processors cause:

(i) receiving, a set of physico-chemical properties $X$ derived from chemical structures of drugs and drug like chemical compounds, a biological response $Y$ associated thereof and a size of variables set $r$ ;

(ii) initializing a population $S_{pop}$ further comprising matrix subsets $\{X_1, X_2, ... X_{pop}\}$ of variables selected from a filtered set of variables $X_f$ of size $n$, wherein $X_f$ is derived from a set of variables $X$, wherein $S_{pop}$ is initialized based on one or more pre-defined criteria, wherein pop represents size of the population, and wherein each matrix subset $X_i$ from $S_{pop}$ comprises variables that are unique from each other and is of the size of variables set $r$ ;

(iii) selecting at least one subset $X_i$ from $S_{pop}$ and at least one path $P_l$ of the at least one selected subset $X_i$, wherein the at least one selected path $P_l$ comprises a variable $v_q$ to be replaced, wherein each variable $v_q$ comprised in the at least one selected path $P_l$ is a vector of size $m$ describing a property of input chemical compounds, and wherein $m$ represents a number of chemical compounds used for building predictive models;

(iv) replacing the variable $v_q$ from at least one subset $X_i$ with remaining $(n - r)$ variables of the set of variables $X_f$ to obtain a set of modified subsets $X_i' \{X_{i1}', X_{i2}', ..., X_{i(n-r)}'\}$, wherein each of the modified subsets $X_{ij}'$ comprises replaced variables for the at least selected path $P_l$, and wherein size of the set of modified subsets $X_i'$ is of $(n - r)$ ;

(v) generating a predictive model for each of the modified subsets $X_{ij}'$ , and calculating an objective function thereof to obtain a first set of predictive models $M_{i,rm}$ and associated objective functions $OF_{i,rm}$, wherein the first set of predictive models $M_{i,rm}$ are generated based on the biological response $Y$ and each of the modified subsets $X_{ij}'$ variable vectors of a set of input chemical compounds ;

(vi) identifying an optimal modified subset of replaced variables $X_i^{optimal}$ from the set of modified subsets $X_i'$ based on an optimal objective function associated with an optimal predictive model $M_i^{optimal}$ being identified from the first set of predictive models $M_{i,rm}$;

(vii) updating the at least one selected path $P_l$ for the optimal modified subset of replaced variables $X_i^{optimal}$, wherein the steps (iv) till (vii) are iteratively performed until one or more predefined criteria are met to obtain an optimal population $S_{pop}^{optimal}$ ;

(viii) identifying an optimal element $X_{optimal}$ of $S_{pop}^{optimal}$ , wherein $X_{optimal}$ comprises an optimal objective function amongst objective functions comprised in other $X_i^{optimal}$ ;

(ix) performing an exhaustive search on a pool of variable $X_{pool}$ created using the optimal population $S_{pop}^{optimal}$ to obtain a set of variable subsets $S_x$, wherein each element of $S_x$ comprises set of $r$ variables;

(x) generating the predictive model for each element of $S_x$ and calculating the objective function thereof to obtain a second set of predictive models $M_x$ and associated objective functions $OF_x$ ;

(xi) identifying, via the one or more hardware processors, a pop number of optimal elements from $S_x$ to update the optimal population $S_{pop}^{optimal}$ and to obtain an updated population $S_{es}$;

(xii) identifying an optimal element $X_{optimal,es}$ amongst elements $X_{es}$ comprised in the updated population $S_{es}$, and comparing an objective function of $X_{optimal,es}$ with an objective function of the identified optimal element $X_{optimal}$ for updation of the identified optimal element $X_{optimal}$;

(xiii) randomly replacing variables of the updated population $S_{es}$ to obtain a perturbed set of population $S_P$; and

(xiv) generating one or more predictive models based on the selected optimal subset of variables $X_{optimal}$.

12. The one or more non-transitory machine-readable information storage mediums of claim 11, wherein the one or more predefined criteria comprise: (i) subsets in which variables having inter correlation amongst each other below a first predefined threshold, (ii) subsets whose variables are correlated with the biological response $Y$ above a second defined threshold or a system generated dynamic threshold; (iii) seeded subsets having variables whose sizes are less than a current subset size and (iv) a user defined preferences for the set of variables X, wherein the at least one selected path $P_l$ is identified based on a relative error value associated with the variable $v_q$, and wherein the steps (ii) till (xiii) are iteratively performed either sequentially or in parallel across multiple processors threads.

13. The one or more non-transitory machine-readable information storage mediums of claim 11, wherein the steps (iv) till (vii) are iteratively performed until the one or more predefined criteria are met, and wherein the one or more predefined criteria further comprise at least one of (a) a predefined number of iterations, (b) frequency of the matrix subset of variables, (c) the optimal objective function reaching a predetermined value, and (d) an improvement in the objective function in subsequent iterations reaching a predefined saturation value or machine epsilon.

14. The one or more non-transitory machine-readable information storage mediums of claim 11, wherein the steps (ix) till (xii) are performed either sequentially or in parallel across multiple processors threads.

15. The one or more non-transitory machine-readable information storage mediums of claim 11, wherein the one or more instructions which when executed by the one or more hardware processors further cause upon satisfying the one or more predefined criteria, and upon iteratively performing the steps (ii) till (xiii), generating a final optimal subset based on the identified optimal element $X_{optimal}$ for predicting biological responses of chemical compounds,

wherein the step of initializing a population $S_{pop}$ comprising matrix subsets $\{X_1, X_2, ... X_{pop}\}$ of variables selected from the filtered set of variables $X_f$ is preceded by transforming the set of variables X to obtain a set of transformed variables; and filtering the set of transformed variables based on one or more statistical criteria and predefined thresholds to obtain a filtered set of variables $X_f$, and wherein the one or more predictive models comprise one or more linear regression models, one or more non-linear regression models, or one or more classification models, and

wherein the one or more predictive models and the identified optimal subset $X_{optimal}$ are used for generating one or more rules or alerts.

SYSTEM
100

MEMORY
102

DATABASE
108

HARDWARE
PROCESSOR(S)
104

INTERFACE(S)
106

**FIG. 1**

Raw Data $X$, $Y(m)$
Desired Subset Size : $r$ —— 202

Filter & Transform Data $X$
Output : $X_f(m, n)$ —— 203

Initialize Population $S_{pop} = \{ X_1, X_2, \ldots, X_{pop}\}$
and identify $X_{optimal}$ —— 204

—— 206

Iterate population
$S_{pop}$

Yes | No

Replacement Module
Input : $X_i$
Output : $X_i{}^{Optimal}$ —— 208

Update $S_{pop}{}^{Optimal}$ with $X_i{}^{Optimal}$

—— 208

Update $X_{Optimal}$ with $S_{pop}{}^{Optimal}$ —— 210

No

Create $X_{pool}$ using $S_{pop}{}^{Optimal}$ —— 212

Exhaustive Search Module
Input : $X_{pool}$
Output : $S_{es}$ —— 214

Random Perturbation
Input : $S_{es}$
Output : $S_p$ —— 216

Termination Condition

Yes

Output : $X_{optimal}$ and
$M_{optimal}$ —— 218

**FIG. 2A**

**Chemical Structures Y(m)**

In house tool

Descriptors /Variables /Features
X — 202

Data Transformation

Data Filteration
$X_f(m, n)$
— 203

Feature Selection using
PERM

Features Selected
$X_{optimal}(m, r)$ — 208

Model $M_{optimal}$ — 218

Non Predicted
Compounds

Predicted Compound
Activities

**FIG. 2B**

receiving a set of physico-chemical properties $X$ derived from chemical structures of drugs and drug like chemical compounds, a biological response $Y$ associated thereof and a size of variables set $r$ — 302

initializing a population $S_{pop}$ comprising matrix subsets $\{X_1, X_2, \ldots X_{pop}\}$ of variables selected from a filtered set of variables $X_f$ of size $n$, wherein $X_f$ is derived from a set of variables $X$, wherein $S_{pop}$ is initialized based on one or more pre-defined criteria, wherein $pop$ represents size of the population, and wherein each matrix subset $X_i$ from $S_{pop}$ comprises variables that are unique from each other and is of size $r$ — 304

selecting at least one subset $X_i$ from $S_{pop}$ and at least one path $P_l$ of the at least one selected subset $X_i$, wherein the at least one selected path $P_l$ comprises a variable $v_q$ to be replaced, wherein each variable $v_q$ is a vector of size $m$ describing a property of input chemical compounds, and wherein $m$ represents the number of chemical compounds used for building predictive models — 306

replacing the variable $v_q$ from at least one subset $X_i$ with remaining $(n - r)$ variables of the set of variables $X_f$ to obtain a set of modified subsets $X_i'$ $\{X_{i1}', X_{i2}', \ldots, X_{i(n-r)}'\}$, wherein each of the modified subsets $X_{ij}'$ comprises replaced variables for the at least selected path $P_l$, and wherein size of the set of modified subsets $X_i'$ is of $(n - r)$ — 308

generating a predictive model for each of the modified subsets $X_{ij}'$, and calculating an objective function thereof to obtain a first set of predictive models $M_{i,rm}$ and associated objective functions $OF_{i,rm}$, wherein the first set of predictive models are generated based on the biological response $Y$ and each of the modified subsets $X_{ij}'$ variable vectors of a set of input chemical compounds — 310

identifying an optimal modified subset of replaced variables $X_i^{optimal}$ from the set of modified subsets $X_i'$ based on an optimal objective function associated with an optimal predictive model $M_i^{optimal}$ being identified from the set of predictive models $M_{i,rm}$ — 312

updating the at least one selected path $P_l$ for the optimal modified subset of replaced variables $X_i^{optimal}$, wherein the steps (iv) till (vii) are iteratively performed until one or more predefined criteria are met to obtain an optimal population $S_{pop}^{optimal}$ — 314

$\text{A}$

**FIG. 3A**

identifying an optimal element $X_{optimal}$ of $S_{pop}^{optimal}$, wherein $X_{optimal}$ comprises an optimal objective function amongst objective functions comprised in other $X_i^{optimal}$ ~~316

performing an exhaustive search on a pool of variable $X_{pool}$ created using the optimal population $S_{pop}^{optimal}$ to obtain a set of variable subsets $S_x$, wherein each element of $S_x$ comprises set of $r$ variables ~~318

generating the predictive model for each element of $S_x$ and calculating the objective function thereof to obtain a second set of predictive models $M_x$ and associated objective functions $OF_x$ ~~320

identifying a $pop$ number of optimal elements from $S_x$ to update the optimal population $S_{pop}^{optimal}$ and to obtain an updated population $S_{es}$; ~~322

identifying an optimal element $X_{optimal,es}$ amongst elements $X_{es}$ comprised in $S_{es}$, and comparing an objective function of $X_{optimal,es}$ with an objective function of the identified optimal element $X_{optimal}$ for updation of the identified optimal element $X_{optimal}$ ~~324

randomly replacing variables of the updated population $S_{es}$ to obtain a perturbed set of population $S_P$ ~~326

generating one or more predictive models based on the selected optimal subset of variables $X_{optimal}$ ~~328

## FIG. 3B

Input : $X_i$

Select one or more paths $P_l$
where $P_l : 1 \le l \le r$

For each $v_q$ associated with $P_l$, replace with $\{v_1, v_2, .. v_n\}$
Output : $X_i'\{X_{i1}', X_{i2}', \ldots X_{i(n-r)}'\}$

Generate Model $M_{i,rm}$

Identify $X'_{ik}$ such that $O.F_{rm}(M'_{ik}) >$
$O.F_{rm}(M'_{ij}) \ \forall \ 1 \le j \le (n-r)$ and $j \ne k$

Update $X_i^{Optimal}$ with $X'_{ik}$

Termination
Condition ?

Yes → Output: $X_i^{Optimal}$

No

Update $P_l$'s using predefined conditions

FIG. 4A

Input : $X_i$, T(no. of threads)

Select one or more paths $P_l$ where $P_l : 1 \leq l \leq r$

For each $v_q$ associated with $P_l$, replace with $\{v_1, v_2, \ldots, v_n\}$ Load (L) = n/T

Thread $T_1$          Thread T2          Thread $T_T$

* * *

Iterate through load (L)

Yes

Generate Model $M_{i,rm}$

No

A

Identify $X'_{ik}$ such that $O.F_{rm}(M'_{ik}) > O.F_{rm}(M'_{ij})$ $\forall$ $1 \leq j \leq L$ and $j \neq k$

Update $X_{iT}^{Optimal}$ with $X'_{ik}$

A          A

Perform reduction and update $X_i^{Optimal}$ with $X_{iT}^{Optimal}$ across all the threads.

Termination Condition ?          Yes

No          Output : $X_i^{Optimal}$

Update $P_l$'s using predefined conditions

FIG. 4B

33

**FIG. 5A**

Input : *Xpool*, Total
Combinations *Tc*, *T* (Threads)

Load balancing across threads
L = Tc/T

Thread( $T_1$ )  Thread( $T_2$ )  Thread( $T_T$ )

Itertate
through load

B

Yes

Uranking the subset

Build the model for
generated subset

No

* * *

B  B

Update the $X_{esT}^{Optimal}$  Update the $X_{esT2}^{Optimal}$  Update the $X_{esTT}^{Optimal}$

Perform reduction across all
threads to update $X_{optimal}$

Output : $X_{optimal}$

FIG. 5B

Input : $S_{es} = \{X_{es1}, X_{es2}, \ldots, X_{esp}\}$

Update $X_{optimal}$ with $X_{Optimal,es}$

Iterate $S_{es}$ ?

Yes → Output : $S_p$

No

Randomly modify $X_{esi}$ to $X'_{esi}$

Update $S_{es}$ to obtain $S_p$

FIG. 6

Europäisches
Patentamt

European
Patent Office

Office européen
des brevets

## EUROPEAN SEARCH REPORT

Application Number

EP 22 20 8797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/144652 A1 (RAMAMURTHI NARAYANAN [IN] ET AL) 24 May 2018 (2018-05-24) * abstract; figures 2,4-6 * * paragraphs [0002] - [0013], [0040], [0044], [0052], [0065] * | 1-15 | INV. G06N20/00 G06N3/086 |
| X | US 2017/364809 A1 (RAMAMURTHI NARAYANAN [IN] ET AL) 21 December 2017 (2017-12-21) * abstract; figures 2,3 * * paragraphs [0002] - [0005], [0028] - [0035] * | 1-15 | |

TECHNICAL FIELDS
SEARCHED      (IPC)

G06N

The present search report has been drawn up for all claims

| Place of search | Date of completion of the search | Examiner |
|---|---|---|
| The Hague | 31 July 2023 | Houtgast, Ernst |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...................................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 20 8797

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2023

| Patent document cited in search report | Publication date | Patent family member(s) | Publication date |
|---|---|---|---|
| US 2018144652 A1 | 24-05-2018 | EP 3324346 A1<br>US 2018144652 A1 | 23-05-2018<br>24-05-2018 |
| US 2017364809 A1 | 21-12-2017 | EP 3258388 A1<br>US 2017364809 A1 | 20-12-2017<br>21-12-2017 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• IN 202221014386